# EUROPÄISCHE PATENTANMELDUNG

(11) **EP 1 657 551 A1**
(43) Veröffentlichungstag der Anmeldung: **17.05.2006**
(21) Anmeldenummer: 04026815.3
(22) Anmeldetag: 11.11.2004
(51) Int. Cl.: G01N 33/94, G01N 33/543

(54) **Verfahren und Kit zum Nachweis von Cannabinoiden**

(71) Anmelder: Envitec-Wismar GmbH, 23966 Wismar (DE)
(72) Erfinder: Scharnagl, Matthias, 23968 Wismar (DE); Fellner, Stephan, Dr., 23968 Wismar (DE); Voss, Silvia, 23970 Wismar (DE); Lindner, Bernd, 23626 Ratekau (DE)
(74) Vertreter: Teipel, Stephan

(57) **Zusammenfassung**

Die vorliegende Erfindung betrifft ein Verfahren und einen Kit zum Nachweis von Cannabinoiden mittels eines Teststreifens.

## Beschreibung

Die vorliegende Erfindung betrifft ein Verfahren und einen Kit zum Nachweis von Cannabinoiden mittels eines Teststreifens.

Teststreifen, insbesondere (immuno)chromatographische Teststreifen (lateral flow immunoassay) für verschiedenste Analyten sind bekannt. Entsprechende Teststreifen eignen sich beispielsweise zum Nachweis von Drogen oder Drogenrückständen in Speichel-, Urin- oder Schweißproben von Testpersonen.

Cannabinoide sind die Zielanalyte bei Speichel- und Schweiß-Tests auf Cannabis. Teststreifen für Cannabinoide sind bekannt, jedoch ist die Sensitivität dieser Teststreifen zu gering, um mit ihnen einen Vor-Ort Test auf Cannabinoide in Speichel oder Schweiß durchführen zu können.

Eine Aufgabe der vorliegenden Erfindung bestand somit darin, ein Verfahren zum Nachweis von Cannabinoiden mittels eines Teststreifens zur Verfügung zu stellen, dass die Nachteile entsprechender Verfahren des Standes der Technik überwindet. Insbesondere soll das Verfahren eine so niedrige Sensitivitätsgrenze aufweisen, dass es sich für Vor-Ort Tests von beispielsweise Schweiß- oder Speichelproben auf Cannabinoide eignet.

Es wurde nun überraschend gefunden, dass die Sensitivitätsgrenze bekannter Teststreifen für Cannabinoide signifikant herabgesetzt werden kann, wenn zusätzlich zu der Probe ein Kohlenhydrat oder ein Derivat davon auf den Teststreifen aufgebracht wird.

Die vorliegende Erfindung betrifft somit ein Verfahren zum Nachweis von Cannabinoiden in einer Probe, wobei die Probe auf einen Teststreifen für Cannabinoide aufgebracht wird, dadurch gekennzeichnet, dass der Teststreifen ein Kohlenhydrat oder ein Derivat davon umfasst oder gleichzeitig mit oder zusätzlich zu der Probe ein Kohlenhydrat oder ein Derivat davon auf den Teststreifen aufgebracht wird.

Bei einem Schnelltest auf Cannabis dienen Cannabinoide als Zielanalyte. Diese Cannabinoide sind vorzugsweise ausgewählt aus der Gruppe, bestehend aus Δ⁹-Tetrahydrocannabinol, Δ⁸⁻Tetrahydrocannabinol, 11-Nor-9-carboxy-Δ⁹-tetrahydrocannabinol, 11-Hydroxy-Δ⁹⁻tetrahydrocannabinol, Cannabidiol und Cannabinol. Besonders bevorzugt werden entsprechende Proben auf Δ⁹-Tetrahydrocannabinol (Δ⁹-THC) analysiert.

Als Teststreifen eignen sich beispielsweise chromatographische Teststreifen, insbesondere immunochromatographische Teststreifen.

Teststreifen zum Nachweis eines oder mehrerer Analyten, wie beispielsweise immunochromatographische Teststreifen (lateral flow immunoassay) sind bekannt. Ensprechende Teststreifen weisen üblicherweise eine Auftragezone auf, auf die die zu untersuchende, insbesondere flüssige Probe aufgetragen wird. Die Probe gelangt von dort in eine Reaktionszone, in der der nachzuweisende Analyt beispielsweise eine Farbreaktion auslöst. Das Auftreten einer Färbung in der Reaktionszone bedeutet dann das Vorliegen eines nachzuweisenden Analyten in der Probe. Alternativ kann aber auch, in Abhängigkeit von der Ausgestaltung des Teststreifens, keine Färbung oder eine geringe Färbung ein positives Testergebnis indizieren.

Bei einem entsprechenden Teststreifen kann es sich beispielsweise um einen immunochromatographischen Teststreifen handeln, bei dem mit kolloidalem Gold gekoppelte Antikörper gegen den entsprechenden Analyten, z. B. Δ⁹-THC, auf ein Pad aufgetrocknet sind. In der Laufstrecke des Teststreifens sind Konjugate aus Analyt und Protein als Linie aufgetrocknet. Die Probe wird gleichzeitig mit oder vor einem Lösungsmittel in Laufrichtung vor oder auf das Samplepad aufgebracht. Das Lösungsmittel dient dann als Laufmittel, welches die Probe verdünnt und kontinuierlich und kontrolliert dem Teststreifen zuführt. Durch das Laufmittel wandert die Probe über die aufgetrocknete Linie des Analyt-Protein-Konjugats und löst dort, falls kein Analyt in der Probe vorhanden ist, die vorerwähnte Farbreaktion aus. Diese Farbreaktion wird jedoch nur verhindert, wenn eine bestimmte Konzentration des Analyten in der Probe bzw. in dem Laufmittel überschritten ist. Die Sensitivität des Tests wird daher durch die Löslichkeit des Analyten in dem Laufmittel bzw. der Probenmatrix begrenzt.

Da Cannabinoide wie Δ⁹-THC in Wasser fast unlöslich sind, bot sich den Erfindern zunächst an, den üblicherweise als Laufmittel verwendeten Puffer (PBS) entweder mit einer lösungsvermittelnden Substanz zu versetzen oder andere Lösungsmittel als Laufmittel zu verwenden, in denen Cannabinoide besser löslich sind. Dementsprechend wurden bekannte lösungsvermittelnde Zusätze wie beispielsweise Rinderserumalbumin (BSA, Bovine Serum Albumin), Human Albumin (HA) und Tween 20 ausgetestet. Es wurde jedoch keine oder eine nur sehr eingeschränkte Verbesserung für die Sensitivität auf Cannabinoide gefunden. Ohne an eine Theorie gebunden sein zu wollen, wird angenommen, dass lösungsvermittelnde Zusätze zwar die Löslichkeit und damit die Konzentration von Cannabinoiden in dem Puffer erhöhen, sich jedoch gleichzeitig negativ auf die Detektion des Analyten auswirken, beispielsweise dadurch, dass sie den Analyten maskieren und so einer Detektion entziehen.

Zudem wurden verschiedene organische Lösungsmittel, wie Ethanol, Propanol, Isopropanol und Butanol, als Laufmittel ausgetestet. Mit diesen Lösungsmitteln wird zwar der Analyt gelöst und in den Teststreifen eingebracht, es zeigte sich jedoch, dass diese Lösungsmittel mit üblichen Teststreifen nicht kompartibel sind, da die für die Auswertung benötigten Kontrollbanden nicht erschienen.

Überraschend haben die Erfinder dann gefunden, dass die Anwesenheit von Kohlenhydraten oder Derivaten davon auf dem Teststreifen zu einer signifikanten Sensitivitätssteigerung beim Nachweis von Cannabinoiden führt, ohne herkömmliche Teststreifen negativ zu beeinflussen.

Das Kohlenhydrat oder ein Derivat davon kann entweder vor Zugabe der Probe auf dem Teststreifen vorhanden sein, beispielsweise indem eine kohlenhydrathaltige Lösung auf den Teststreifen aufgetrocknet wird. In diesem Fall wird die Probe entweder direkt oder nach Vermischen mit einem Lösungsmittel wie einem Puffer auf die kohlenhydrathaltige Auftragezone aufgebracht. Alternativ kann das Kohlenhydrat oder ein Derivat davon gleichzeitig mit der Probe auf den Teststreifen aufgebracht werden. Dies kann beispielsweise dadurch geschehen, dass die Probe vor Aufbringen auf den Teststreifen mit dem Kohlenhydrat entweder direkt oder beispielsweise als Kohlenhydratlösung vermischt wird. Schließlich kann das Kohlenhydrat oder ein Derivat davon zusätzlich zu der Probe auf den Teststreifen aufgebracht werden. Dies kann entweder zeitlich vor oder nach dem Aufbringen der Probe erfolgen. Das Kohlenhydrat kann dabei entweder direkt oder als Lösung entweder auf die Auftragezone der Probe oder in Laufrichtung vor die Auftragezone auf den Teststreifen aufgebracht werden.

Als Kohlenhydrate und Kohlenhydratderivate eignen sich beispielsweise Mono-, Oligo- und Polysaccharide und deren Derivate sowie Verbindungen, die Kohlenhydrate kovalent gebunden, beispielsweise an Lipide oder Eiweiße, enthalten. Bevorzugte Kohlenhydrate und Kohlenhydratderivate sind Zucker und Zuckerderivate, insbesondere Cyclodextrine, Cyclodextrinderivate, Amylose, Zellulose, Glykogen, Saccharose, Glukose, Fruktose, Hydroxypropylmethylcellulose sowie Mischungen hieraus. Bevorzugt werden Cyclodextrine und Cyclodextrinderivate, wie β-Hydroxypropylcyclodextrin (2-Hydroxypropylcycloheptaamylose), β-Methylcyclodextrin (Methylcycloheptyamylose), γ-Hydroxypropylcyclodextrin (2-Hydroxypropylcyclooctaamylose), α-Cyclodextrin (Cyclohexaamylose), β-Cyclodextrin (Cycloheptaamylose), γ-Cyclodextrin (Cyclooctaamylose), Carboxyethylcyclodextrin, Carboxymethylcyclodextrin, Maltosylcyclodextrin, Glycosylcyclodextrin, Hydroxyethylcyclodextrin, Cyclodextrin-Dimere, Cyclodextrin-Trimere, Cyclodextrin-Polymere und Mischungen hieraus. Besonders bevorzugt wird β-Hydroxypropylcyclodextrin. Die Cyclodextrine und Cyclodextrinderivate können natürlichem oder synthetischem Ursprung sein.

Entsprechende Cyclodextrine und deren Derivate sind beispielsweise von der Firma Wacker kommerziell erhältlich. Als Cyclodextrinderivate eignen sich beispielsweise Methyl-, Hydroxypropyl-, Hydroxyethyl-, Sulfobutyl-, Acetyl- Carboxyethyl-, Carboxymethyl-, Maltosyl-, Glycosyl- und verzweigte Cyclodextrine. Weitere geeignete Cyclodextrinderivate sowie Cyclodextrin-Dimere, -Trimere und -Polymere sind in der EP-B-1 183 538 beschrieben, deren Inhalt daher in die vorliegende Anmeldung aufgenommen wird.

In einer Ausführungsform der vorliegenden Erfindung wird das Kohlenhydrat oder Kohlenhydatderivat als Lösung, insbesondere als wässrige PBS-Puffer-Lösung auf den Teststreifen aufgebracht. Dieser Puffer kann vor Zugabe des Kohlenhydrats oder Kohlenhydatderivats einen pH-Wert von beispielsweise etwa 7,4 aufweisen.

Das Kohlenhydrat oder Kohlenhydatderivat kann in der Lösung in einer Konzentration im Bereich von 0,1-5000 g/l, beispielsweise 1-2500 g/l, insbesondere 30-1000 g/l, bevorzugt 100-300 g/l, mehr bevorzugt 150-250 g/l und am bevorzugtesten etwa 200 g/l, bezogen auf die Gesamtlösung, vorliegen.

Die Probe kann vor oder nach der Lösung auf den Teststreifen aufgebracht werden. Vorzugsweise wird die Probe jedoch zunächst mit der Lösung gemischt und anschließend mit dem Teststreifen in Kontakt gebracht. Alternativ kann die Lösung auf den Teststreifen aufgetrocknet und später die Probe aufgebracht werden.

In einer besonders bevorzugten Ausführungsform der vorliegenden Erfindung handelt es sich bei der Probe um eine Schweiß- oder Speichelprobe, insbesondere eine Schweißprobe. Diese kann beispielsweise dadurch genommen werden, dass ein Probennehmer (z. B. ein Schwamm) mit einer geringen Menge der Lösung benetzt und über Stirn, Achselhöhle, die Haut hinter dem Ohr, etc. eines Probanden gewischt wird. Durch den Hautkontakt wird eine Schweißprobe auf den Probennehmer übertragen. Dann wird zusätzliche Lösung auf den Probennehmer gegeben, um die Probe zu verdünnen. Anschließend wird die Probe beispielsweise durch Ausdrücken des Probennehmers auf den Teststreifen übertragen. Für die Durchführung dieses Tests eignet sich in besonderer Weise die in der WO 02/097389 beschriebene Vorrichtung zum Sammeln flüssiger Proben, die neben dem Probennehmer-Schwamm den Teststreifen sowie eine Auspressvorrichtung zum Auspressen des Schwamms auf den Teststreifen umfasst. Die Vorrichtung kann dabei einen oder mehrere Teststreifen umfassen, wobei jeder Teststreifen für den Nachweis von einem oder mehreren Analyten geeignet sein kann. Diese Vorrichtung in Verbindung mit dem erfindungsgemäßen Verfahren ermöglicht einen Drogenschnelltest auf Cannabinoide, der aufgrund der Senkung der Sensitivitätsgrenze für Vor-Ort Tests auf Cannabis eine deutliche Verbesserung darstellt.

Durch die Anwesenheit von Kohlenhydraten und deren Derivaten beim Nachweis von Cannabinoiden mittels eines Teststreifens wird die Sensitivität dieses Test signifikant erhöht.

Daher betrifft die vorliegende Erfindung auch die Verwendung von Kohlenhydraten und deren Derivaten zur Erhöhung der Sensitivität von Teststreifen auf Cannabinoide.

Die vorliegende Erfindung betrifft ferner einen Kit zum Nachweis von Cannabinoiden in einer Probe, umfassend einen Teststreifen für Cannabinoide und ein Kohlenhydrat oder ein Derivat davon. Vorteilhafterweise umfasst der Kit eine vorbestimmte Menge einer Lösung des Kohlenhydrats oder eines Derivats davon. Beispielsweise kann der Kit die aus der WO 02/097389 bekannte Vorrichtung zum Sammeln flüssiger Proben einschließlich eines Teststreifens für Cannabinoide zusammen mit einer Ampulle enthalten, die die vorbestimmte Menge der Kohlenhydratlösung bzw. Kohlenhydratderivatlösung enthält. Beispielsweise können in der Pufferampulle ca. 0,9 ml Lösung semisteril eingeschweißt sein. Alternativ kann eine Kohlenhydratlösung oder eine Kohlenhydratderivatlösung auf dem Teststreifen aufgetrocknet sein. In einer weiteren alternativen Ausführungsform kann sich die Ampulle mit der Kohlenhydratlösung bzw. Kohlenhydratderivatlösung in der Vorrichtung zum Sammeln flüssiger Proben befinden, wo sie beim Schließen dieser Vorrichtung automatisch, beispielsweise durch Zerstechen geöffnet wird, und die Lösung so automatisch auf den Teststreifen gelangt.

Das nachfolgende Beispiel soll die vorliegende Erfindung veranschaulichen, ohne sie jedoch hierauf einzuschränken.

### Beispiel

In dem vorliegenden Beispiel wurde PBS-Puffer bzw. Wasser mit verschiedenen lösungsvermittelnden Zusätzen versehen und die Sensitivitätsgrenze von herkömmlichen immunochromatographischen Teststreifen für Δ⁹-THC wurde bestimmt. Die Ergebnisse dieser Untersuchungen sind in der nachfolgenden Tabelle 1 zusammengefasst.

**Tabelle 1**

| Lösungsmittel | Zusatz | Sensitivitätsgrenze Δ⁹-THC |
|---|---|---|
| PBS-Puffer | Ohne Zusatz | > 5000 ng/ml |
| PBS-Puffer | 20 % β-Hydroxypropylcyclodextrin | 250 ng/ml |
| PBS-Puffer | 0,5 % BSA (Bovine Serum Albumin) | > 1000 ng/ml |
| PBS-Puffer | 0,5 % HA (Human Albumin) | > 1000 ng/ml |
| H₂O | 0,15 % Tween 20 | > 1000 ng/ml |

Die vorstehenden Testergebnisse zeigen, dass BSA, HA und Tween 20 die Sensitivitätsgrenze des Teststreifens nicht signifikant herabsetzen. Erst durch den Zusatz von β-Hydroxypropylcyclodextrin wird der cut-off für das Δ⁹-THC noch einmal um den Faktor 4 gesenkt.

In einem weiteren Beispiel wurden Ethanol, Propanol, Isopropanol und Butanol als Lösungsmittel eingesetzt. Es zeigte sich jedoch, dass die Teststreifen mit diesen Lösungsmitteln instabil waren. Die Kontrollbanden erschienen nicht.

## Patentansprüche

1. Verfahren zum Nachweis von Cannabinoiden in einer Probe, wobei die Probe auf einen Teststreifen für Cannabinoide aufgebracht wird, **dadurch gekennzeichnet, dass** der Teststreifen ein Kohlenhydrat oder ein Derivat davon umfasst oder gleichzeitig mit oder zusätzlich zu der Probe ein Kohlenhydrat oder ein Derivat davon auf den Teststreifen aufgebracht wird.

2. Verfahren nach Anspruch 1, **dadurch gekennzeichnet, dass** das Kohlenhydrat oder ein Derivat davon ein Zucker oder Zuckerderivat ist, vorzugsweise ausgewählt, aus der Gruppe, bestehend aus Cyclodextrinen, Cyclodextrinderivaten, Amylose, Zellulose, Glykogen, Saccharose, Glukose, Fruktose, Hydroxypropylmethylcellulose und Mischungen hieraus.

3. Verfahren nach Anspruch 2, **dadurch gekennzeichnet, dass** das Cyclodextrin bzw. das Cyclodextrinderivat ausgewählt ist aus der Gruppe bestehend aus β-Hydroxypropylcyclodextrin, β-Methylcyclodextrin, γ-Hydroxypropylcyclodextrin, α-Cyclodextrin, β-Cyclodextrin, γ-Cyclodextrin, Carboxyethylcyclodextrin, Carboxymethylcyclodextrin, Maltosylcyclodextrin, Glycosylcyclodextrin, Hydroxyethylcyclodextrin, Cyclodextrin-Dimeren, Cyclodextrin-Trimeren, Cyclodextrin-Polymeren und Mischungen hieraus.

4. Verfahren nach einem der vorhergehenden Ansprüche, **dadurch gekennzeichnet, dass** das Kohlenhydrat β-Hydroxypropylcyclodextrin ist.

5. Verfahren nach einem der vorhergehenden Ansprüche, **dadurch gekennzeichnet, dass** der Teststreifen das Kohlenhydrat oder ein Derivat davon enthält.

6. Verfahren nach einem der Ansprüche 1-4, **dadurch gekennzeichnet, dass** das Kohlenhydrat oder ein Derivat davon als Lösung auf den Teststreifen aufgebracht wird.

7. Verfahren nach Anspruch 6, **dadurch gekennzeichnet, dass** die Lösung einen wässrigen PBS-Puffer umfasst.

8. Verfahren nach Anspruch 6 oder 7, **dadurch gekennzeichnet, dass** das Kohlenhydrat oder ein Derivat davon in der Lösung in einer Konzentration im Bereich von 0,1-5000 g/l, vorzugsweise 100-300 g/l und am bevorzugtesten etwa 200 g/l, bezogen auf die Gesamtlösung, vorliegt.

9. Verfahren nach einem der vorhergehenden Ansprüche, **dadurch gekennzeichnet, dass** der Teststreifen ein chromatographischer, vorzugsweise ein immunochromatographischer Teststreifen ist.

10. Verfahren nach einem der vorhergehenden Ansprüche, **dadurch gekennzeichnet, dass** die Probe eine Schweiß- oder Speichelprobe ist.

11. Verfahren nach einem der Ansprüche 6-10, **dadurch gekennzeichnet, dass** die Probe mit der Lösung gemischt und anschließend mit dem Teststreifen in Kontakt gebracht wird.

12. Verwendung von Kohlenhydraten oder Derivaten davon zur Erhöhung der Sensitivität von chromatographischen Teststreifen auf Cannabinoide.

13. Kit zum Nachweis von Cannabinoiden in einer Probe, umfassend einen Teststreifen für Cannabinoide und ein Kohlenhydrat oder ein Derivat davon.

14. Kit nach Anspruch 13, umfassend eine vorbestimmte Menge einer Lösung des Kohlenhydrats oder eines Derivats davon.
